# EUROPEAN PATENT APPLICATION

(11) **EP 0 587 205 A1**
(43) Date of publication of application: **16.03.1994**
(21) Application number: 93202102.5
(22) Date of filing: 19.07.1993
(51) Int. Cl.: C12N 5/00

(54) **Culture system for anchorage dependent cells**

(30) Priority: 23.07.1992 EP 92202261
(71) Applicant: DUPHAR INTERNATIONAL RESEARCH B.V, NL-1380 AC Weesp (NL)
(72) Inventor: Brands, Ruud, NL-1380 AC Weesp (NL); Snoek, Marja C., NL-1380 AC Weesp (NL)
(74) Representative: Breepoel, Peter M.

(57) **Abstract**

The present invention is concerned with solid carriers for the attachment of cells which are coated with a polypeptide (preferably of between 5 and 25 amino acids) containing the amino acid sequence Arg-Gly-Asp.
The attachment of cells to these carriers is fast and the attached cells reach confluency very rapidly.
A further advantage is that detachment of the cells from the carriers can be affected by simply lowering the pH to a value below 7.0.

SEQUENCE LISTING

(1) GENERAL INFORMATION:

(i) APPLICANT:
(A) NAME: Duphar International Research B.V.
(B) STREET: C.J. van Houtenlaan 36
(C) CITY: WEESP
(E) COUNTRY: The Netherlands
(F) POSTAL CODE (ZIP): 1381 CP
(G) TELEPHONE: (0)2940-77352
(H) TELEFAX: (0)2940-80253
(I) TELEX: 14232

(ii) TITLE OF INVENTION: Culture system for anchorage dependent cells

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 5 amino acids
(B) TYPE: amino acid
(D) TOPOLOGY: linear

(ii) MOLECULE TYPE: peptide

(v) FRAGMENT TYPE: internal

(ix) FEATURE:
(A) NAME/KEY: Peptide
(B) LOCATION: 1
(D) OTHER INFORMATION: /label= N-terminal
/note= "amino acid or polypeptide of up to 21
amino acids"

(ix) FEATURE:
(A) NAME/KEY: Peptide
(B) LOCATION: 5
(D) OTHER INFORMATION: /label= C-terminal
/note= "amino acid or polypeptide of up to 21
amino acids"

(xi) SEQUENCE DESCRIPTION: SEQ ID NO: 1:

Xaa Arg Gly Asp Xaa
1               5

(2) INFORMATION FOR SEQ ID NO: 2:

(i) SEQUENCE CHARACTERISTICS:
(A) LENGTH: 6 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO: 2:

Lys Gly Arg Gly Asp Ser
1               5

(2) INFORMATION FOR SEQ ID NO: 3:

(i) SEQUENCE CHARACTERISTICS:
(A) LENGTH: 5 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO: 3:

Gly Arg Gly Asp Ser
1               5

## Description

The present invention is concerned with a solid carrier suitable for the attachment of cells, with a method for the preparation thereof and with methods for the use of these solid carriers in the culturing and harvesting of anchorage dependent cells.

Anchorage dependent cell lines attach to a solid substrate. The actual attachment is mediated by attachment factors that either can be synthesized by certain types of cells themselves (e.g. fibroblasts) or must be provided with the growth medium. One of the rich sources of attachment factors is serum, that is added as additional component to most classical growth media. However, due to the expanding concern for extraneous agents in serum and the varying quality of the different batches, a strong trend towards the use of serum free media is noted, to which additonal purified attachment factors are added if needed for the celltypes under study. These attachment factors, of which fibronectin and vitronectin are most known, all share a common epitope consisting of a sequence of three amino-acids (Arg-Gly-Asp). This epitope is recognized by a receptor (e.g. the fibronectin receptor) on the cell surface and is minimally required for proper attachment. By cross-linking (Arg-Gly-Asp) to a solid substrate, hence, in principle cells can attach.

US Patent No. 4,578,079 describes the use of fibronectin related peptides (containing the sequence Arg-Gly-Asp) in the attachment of cells to a solid substrate. To this end the peptide, which has to contain a COOH-terminal cystine residue, is attached via the heterobifunctional cross-linker SPDP (N-succinidimyl 3-(2-pyridyldithio)propionate) to rabbit IgG which is immobilized to the (plastic) substrate.

However, in view of the increasing requirements as to safety there is a need for a method of attachment of cells, wherein no natural proteins are used. As an alternative to natural proteins poly-L-lysine has been proposed. However, these polyamino acids have the property to bind to any negatively charged protein. Hence, these are not suited for specific binding of cells. Any protein - also any protein from the culture medium - will be bound to the poly-L-lysine. Therefore the specific binding of cells is less efficient.

Furthermore, for efficiency reasons there is a need to make the period needed to establish adherence of the cells to the solid substrate as brief as possible.
Accordingly, the present invention is concerned with a solid carrier suitable for the attachment of cells essentially consisting of a solid substrate and polypeptide molecules containing the amino acid sequence Arg-Gly-Asp attached thereto which is characterized in that the polypeptide molecule is directly bound to the solid substrate by a non-covalent linkage at a pH above 7.0.

Preferably, said polypeptide molecules contain 5-25 amino acids and have the general formula 1 (also included as SEQ ID NO:1)

Xaa-Arg-Gly-Asp-Xaa (1)

wherein both symbols Xaa represent an amino acid or a polypeptide containing up to 21 amino acids.

Preferred polypeptides according to the invention have 5-7 amino acids. More in particular said polypeptide molecules have the amino acid sequence of formula 2 (also included as SEQ ID NO: 2)

Lys-Gly-Arg-Gly-Asp-Ser (2)

For many purposes there is a need to detach the cells from the solid substrate after adequate cell growth. This detachment generally results in considerable losses of cells and/or puitative damage to surface proteins and involves the need of expensive reagents, which are difficult to recycle. At present, anchorage-dependent cells are detached by the use of proteolytic enzymes, for example trypsin, at about neutral pH.

Surprisingly it has been found that the method of culturing anchorage dependent cells according to the present invention can be followed by a much more simple method of detachment.

Accordingly, another aspect of the present invention resides in an easy and safe method for the detachment of cells bound with the use of the peptides according to the invention. To this end the pH of the medium surrounding the cells attached according to the invention can be lowered to a value below 7.0. Advantageously the pH of the medium is not below 6.2. Furthermore, in order to promote the detachment a proteolytic enzyme, such as trypsin can be added to the medium. However, in exploiting the peptides according to the present invention much less proteolytic enzyme activity is needed to detach the cells from the solid substrate. In case trypsin is used, it is an advantage that at the pH below 7 the activity of the proteolytic enzyme will be substantially less than at its optimal pH of about 8. Thus the amount of damage to the cell proteins is considerably reduced.

Advantageously, for this application the polypeptide according to formula I contains one or more amino acids which have within the peptide an apparent pKₐ value in the range of 6.8-7.2, such as histidine.

Optionally, the polypeptide molecules of the formula 1 can be cross-linked, with the solid substrate e.g. using a bifunctional cross-linking agent such as SPDP, or e.g. an aldehyde such as formaldehyde or glutaraldehyde.

Suitable solid substrates according to the present invention are made of glass (e.g. flasks), or of plastics or other synthetic materials (e.g. microcarriers or macrocarriers)

The present invention is also concerned with a method for the preparation of a solid carrier suitable for the attachment of cells comprising contacting a polypeptide molecule of formula 1 with a solid substrate at a pH above 7.0 under conditions preventing a covalent linking of the polypeptide molecule to the solid substrate.

Optionally, this non-covalent linking can be followed by a cross-linking reaction between the polypeptide molecules and the solid substrate using a cross-linking agent as described above.

As a result the present invention provides also for a method for the culturing of anchorage dependent cells wherein said cells are contacted with a solid carrier according to the present invention and subsequently incubated in a suitable medium under growth promoting conditions at a pH above 7.0.

This culturing method facilitates the subsequent obtention of a suspension of said anchorage dependent cells by simply lovering the pH of the growth medium to a pH below 7.0 and subsequent separation of the cells from the solid carrier.

The present invention can be illustrated by the following examples.

### EXAMPLE 1

### Attachment of cells to carrier

T flasks were coated overnight with the pentapeptide (Gly-Arg-Gly-Asp-Ser; also included as SEQ ID NO: 3) and the hexapeptide (Lys-Gly-Arg-Gly-Asp-Ser), respectively, by incubation at room emperature, at pH values between 5.0-7.1.

Subsequently, the coated T flasks were incubated with MDCK cells during six hours at 37°C, at pH values between 7.0 and 7.4.

The T-flasks coated at pH values above 5.5 all showed complete confluency with both peptides.

The T-flasks coated at pH values below 5.5 showed 60-70% confluency.

As a control, uncoated T-flasks were incubated with MDCK cells. Under these conditions attachment of cells was very significantly less than for the peptide coated flasks.

### EXAMPLE 2

### Detachment of cells from a peptide-coated carrier

The peptide- and cell-coated T-flasks of EXAMPLE 1 were subsequently treated with solutions of various pH values below 7.4.

The cells bound to T-flasks coated with the hexapeptide showed detachment characteristics (rounding off) at pH below 6.5.

The cells to bound T-flasks coated with the pentapeptide showed detachment characteristics at pH below 6.0.

Cells bound to uncoated T-flask showed much less pronounced detachment characteristics from coated T-flasks could be completely detached by shear stress or by treatment with trypsin at much lower concentrations than for cells at uncoated T-flasks.

## Claims

1. Solid carrier suitable for the attachment of cells essentially consisting of solid substrate and a polypeptide molecule containing the amino acid sequence Arg-Gly-Asp attached thereto characterized in that the polypeptide molecule is directly bound to the solid substrate by a non-covalent linkage.

2. Solid carrier according to claim 1 characterized in that the polypeptide molecule contains 5-25 amino acid.

3. Solid carrier according to claim 1-2 characterized in that the polypeptide molecules are cross-linked with the carrier using a cross-linking agent.

4. Method for the preparation of a solid carrier suitable for the attachment of cells comprising contacting a polypeptide molecule containing the amino acid sequence Arg-Gly-Asp with a solid substrate under conditions preventing a covalent linking of the polypeptide molecule to the solid substrate.

5. Method for the preparation of a solid carrier suitable for the attachment of cells wherein the method of claim 4 is followed by a cross-linking reaction between the polypeptide molecules and the solid substrate using a cross-linking agent.

6. Method for the culturing of anchorage dependent cells wherein said cells are contacted with a solid carrier according to claim 1-3 and subsequently incubated in a suitable medium under growth promoting conditions at a pH above 7.0.

7. Method for the release of anchorage dependent cells from a solid substrate on which they are grown wherein the method according to claim 6 is followed by a lowering of the pH below 7.0 in the presence of trypsin and subsequent harvesting of the cells detached from the solid substrate.
